# EUROPEAN PATENT SPECIFICATION

(11) **EP 1 646 981 B1**
(45) Date of publication and mention of the grant of the patent: **12.01.2011**
(21) Application number: 04727672.0
(22) Date of filing: 15.04.2004
(51) Int. Cl.: A61B 5/103, G06T 7/00

(54) **AUTOMATIC DETECTION OF SKIN LESIONS**
AUTOMATISCHE ERMITTLUNG VON HAUTANOMALIEN
DETECTION AUTOMATIQUE DES LESIONS DE LA PEAU

(30) Priority: 22.04.2003 IT RM20030184
(43) Date of publication of application: 19.04.2006
(73) Proprietor: DSC S.r.l., 00154 Roma (IT)
(72) Inventor: MELCHI, Carmelo, Francesco, I-00167 Roma (IT); BELLERINO, Oscar, I-00040 Pomezia-(RM) (IT)
(74) Representative: Sarpi, Maurizio
(86) International application number: PCT/IT2004/000217
(87) International publication number: WO 2004/095372

(56) References cited:
- WO-A-00/76398
- WO-A-02/33649
- WO-A-99/59106
- DE-U- 20 010 292
- US-A- 5 836 872

## Description

The present invention relates to the secondary prophylaxis of a neoplasm which originates from the dermal pigmentary system as well as the follow-up of the inflammatory and/or degenerative skin diseases, and particularly, a method that shows automatically and eventually any variation in the number and/or morphology of skin lesions of the patient by the automated detection of the state of the skin surface of the patient in following tests and the comparison of the last detection with the preceding one.

Another object of the present invention is an apparatus for carrying out the method. This in order to draw the physician's attention to all and only the skin lesions with dermatological concern, such as nevus, psoriasis, vitiligo, tumours and/or dermal lymphomas, etc, which must be checked by the physician himself. To this end, it should be appreciated that frequent reference is made hereinafter to nevus, however, this has not to be intended as limitative because this invention can be applied, without modifications, to any type of dermal lesions with dermatological concern.

Dermal melanoma is a malignant neoplasm that originates from the pigmentary cells (melanocytes) of epidermis. Its incidence in Italy inferred from the tumour registers varies according to age: 5.04 out of one hundred thousand inhabitants up to 44 years old, 27.3 out of one hundred thousand inhabitants between 65 and 77 years old.

The risk factors singled out for the onset of the disease come from: genetics (familial melanoma, dysplastic nevus syndrome, xeroderma pigmentosus); phenotypes (colour of eyes, colour of hair, phototype, lentigines, number of common nevi, number of atypical nevi); environment (ultraviolet radiations, solar radiations, pesticides, environmental polluting agents).

Evidences has been found that most melanomas do not arise from nevus cells, and although the disease nature history is not well known the development of neoplasm is suspected of being biphasic: after a relative quick growth the melanoma can remain stable as for morphology and growth also for many years. In this phase both physician and patient can hardly suspect its presence as its aspect is not different from an ordinary nevus; quick changes leading usually to the excision of the skin lesion and its histological test would set in only tardily.

The disease prognosis is entirely bound to the thickness of the neoplasm upon its ablation: a survival of 10 years is expected in 98 percent of cases if the.thickness is not greater than 0.4 mm, and 10 percent if the thickness is greater than 1.5 mm. Therefore, the fact that every effort has been made to come to an early diagnose of such disease is not astonishing.

One paradigm of medicine is the diagnosis of the disease as deviation from the normality: therefore, the delineation of structure and function of a sound human organism (normal human anatomy and physiology) is primary for individuating and cataloguing states of alteration (pathologies).

However, the definition of the typical morphology of a benignant melanocytic nevus is very hard: in fact their variability is huge. Dimension, colour, contour, axes of symmetry can show quite unlike characteristics both in the same and different individuals. Furthermore, it's now a sure thing that there is no relation between macroscopic and histological aspects of a nevus: signs of dysplasia can be noted both in morphological atypical nevi and the so-called ordinary nevi which are clinically reassuring.

In addition, melanomas ablated fortuitously or not suspected in advance are described as lesions of the morphology quite similar to nevi or, in contrast, quite different from the pigmentary structures.

The definition of a boundary line between benignant nevi and initial malignant melanomas by exclusively clinic criteria is then a hazardous practice having poor scientific character. In such a situation it is easy to understand that physicians turn preferably to the excision-biopsy of lesions with irregular aspect (according to at least one of the following criteria: A=asymmetry; B=irregular contour; C=variegated colour; D=dimension greater than 5 mm), above all those whose natural history, i.e. date of onset, evolution, stability of the morphological characteristics in time, cannot be reconstructed.

One way of allowing physicians to increase their capability of diagnosing initial melanoma is to monitor subjects exposed to the risk through a photographic check of the pigmented lesions. Another approach is to discriminate nevi and melanomas by using the technology of the digital image analysis (digital epiluminescence).

The self-test of the skin to detect abnormalities or changes plays an important role in the early diagnose of melanoma [1, 2].

The effectiveness of such practice is conditioned, however, by the information service to people about the risk "melanoma", individual socio-economic conditions, and localization of the neoplasm. Epidemiological, experimental studies [3, 4] showed that also careful subjects looking after changes in the dermal state have high difficulties to detect variations in the aspect of the pigmentary lesions, above all because the recollection of the morphology of their own nevi becomes confused and fragmentary with time.

The practice of monitoring subjects exposed to the risk melanoma through the manual collection of photographic documentation of the whole body or lesions suspected of neoplasm is largely in use by dermatologists. In fact it has been proved that the discovery of initial melanomas is made in subjects exposed to a high risk and checked by physicians who use photographic means to be certain of the morphological stability of patients' lesions to the greatest extent and more easily than in subjects who are only checked by a simple clinic skin test.

The photographic check modes are miscellaneous: analogue cameras, digital cameras, digital image collection and storage systems such as those known on the market and in literature by the following names: "Dermagraphix", "Mole Max II", Catia (Computer-Aided Topodermatographic Image Analysis); some of the latter apparatus are also able to carry out tests of single lesions by epiluminescence. The staff involved may be various: physicians, technicians, hospital attendants, professional photographers.

It should be noted, however, that the comparison of the images collected later on is made by the specialist at sight and on the base of his experience to consider any significant modification of the morphologic characteristics of the monitored lesions. The limitations of such practice are not well known. However, it is certain that a standardization of the image collection modes (lighting system, angle shot, shot distance, characteristics of the photographic apparatus used in the different sessions, modes of dividing the photographed body portions) increases the effectiveness of the check method.

Furthermore, the visual comparison of images of pigmented lesions in multi-nevus subjects is particularly hard and expensive.

All of the available technologies for the light sources that can be used (visible light; coherent light; polarized light; interferometry; fluorescence; epiluminescence) have been taken into consideration to identify known concordant reference points relative to the analytical data resulting from the different methods used.

Such considerations and the possible consequences of the use of the different light sources to the operating functionality of the invention brought to the conclusion of implementing an apparatus that uses stable, normalized visible light.

An analytical inquiry into known apparatus aiming at the same purpose as the present invention, in particular methods of digital analysis, methods of image analysis, as well as availability of tools that can be used for the invention, brought only to systems using the epiluminescence technique to detect the malignancy or not of single lesions suspected by the clinic test.

The study of systems for collecting three-dimensional digital images induced the present inventors to reject the use of such methods in the present invention because of the high increase in the difficulty of operation in comparison with the short advantages that could be achieved.

The medical literature teaches essentially that:
- subjects with more than 50 nevi run the risk of melanoma onset and often have also pigmented lesions due to sunburns (solar freckles) as well as other skin lesions not of melanocytic origin;
- melanoma is associated with a pre-existent nevus only in 15% of case, the remaining 85% arising on formerly uninjured skin;
- the modifications of only colour characteristics of a pre-existent lesion can give the premature signal of a canceration in progress;
- the melanocytic nature of a pigmented lesion with a diameter lower than 2 mm is clinically uncertain.

As a result, the detection of the onset of a new pigmentary lesion with a diameter of at least 2 mm and/or the increase in the diameter of one or more pre-existent nevi by at least one mm and/or the variation of their morphology/colour in people exposed to the risk of melanoma are very useful data inducing the patients to be subjected to early diagnostic instrumental tests (epiluminescence) or nature tests (histological sampling), thus achieving prognostic advantages in case of positive result (onset of melanoma).

It is known, from US5836872, a technique wherein different images are recorded by changing the focal depths without modify the relative position of subject and camera.

It is also known, from WO 02/33649, a technique wherein more image of the subject are taken.

The main object of the present invention is then to provide a method and a relative apparatus able to detect and to show eventual modifications in the number and the morphology/colour of skin lesions of patients in a few dozens of minutes in a simple, reliable, fully automatic manner. Such apparatus and method are defined in claims 1 and 11, respectively.

After a thorough analysis of the known technique, a simple USA instrument having three evident purposes has been found on the market, and namely:
1. mapping the nevi in defined areas (counting and numbering them);
2. giving the physician a regular, complete picture of the examined subject's skin;
3. filing general data along with data pointed out by the physician during the visit together with a specialist of video image collection who takes the macro shot of a determined (suspected) nevus falling into a particular quadrant.

Upon the next check, the USA apparatus counts the nevi again and finds new ones, if they appear (however, the minimum dimension threshold is not defined).

The specialist of video image collection takes then a new macro shot of the nevus or nevi under control for the next analysis by the physician so that the system only allows a visual comparison between the preceding and the present states of the nevus or nevi and not an automatic graphic comparison between two images of the same or more nevi detected in subsequent times. However, such system does not fully satisfy the present needs of monitoring and quickly detecting any anomaly or suspected skin lesion. In fact, it cannot check the growth of any clinically insignificant nevus.

Although on one side the performance of this known apparatus is encouraging as it gives evidences of the arisen nevi and compares two images of the same suspected nevus taken with a time interval from each other, on the other side, it is necessary to consider its high operating cost as the presence of a specialist of video image collection to take the macro shot is needed. In addition, it is not possible to automatically detect clinically little evident initial growth or those most significant for a better prognosis.

A second object of the present invention is to overcome the limits mentioned above and to keep the advantages.

A third object of the invention is to provide a system capable of collecting standardized digital images of the whole body and/or a single lesion; to compare automatically the digital images of the same body segment of the same patient detected in subsequent times and then to show to the operator -who should not necessarily be a physician or a specialist of video image collection- modifications of the pre-existent lesions of the body or the onset of a new lesion; and to weigh statistically the probability that the detected modifications can be effective or depending on little random modifications of the mode of detection.

The advantages of the invention are connected to the capability for the digital image analysis of: highlighting structure, dimension or contour modifications of the pigmented lesions monitored by greater reproducibility and precision than the visual comparison; reducing time and cost necessary to compare the aspect in time of each single pigmentary lesion in multi-nevus subjects; giving the tested subject a base documentation of his or her pigmented lesions that can be easily consulted for a more effective self-check.

This has been accomplished according to the invention by providing an automated apparatus that collects and compares the following parameters in order to compare images of the same subject collected in subsequent times to highlight automatically any morphology/colour difference of the lesions under control:
- number of known nevi/number of detected nevi;
- minimum nevus dimensions that can be detected equal to 3 mm²;
- minimum nevus dimension modifications that can be detected equal to 1 mm.

A better understanding of the invention will follow from the following detailed description with reference to the accompanying drawings that show by way of a not limited example a preferred embodiment thereof.

In the drawings:
Figures 1 and 2 are two axonometric views of the structure of the apparatus showing schematically the arrangement of the reference axes and the relative rotation angles of the image collection means, respectively;
Figure 3 is a logical functional block diagram of the apparatus according to the invention;
Figures 4 and 5 are block diagrams relative to the scanning operation (i.e. transfer of processed input data) as well as scanning of parameters of Fig. 3;
Figures 6, 9 and 10 show by way of example the positions of the frames with respect to the body of the patient;
Figure 7 and 8 show an example of the variation of a frame image overlap in case of a dimension modification (increase) with respect to the preceding session;
Figure 11 shows schematically an example of an image collection apparatus provided with distance sensors and laser beam projector;

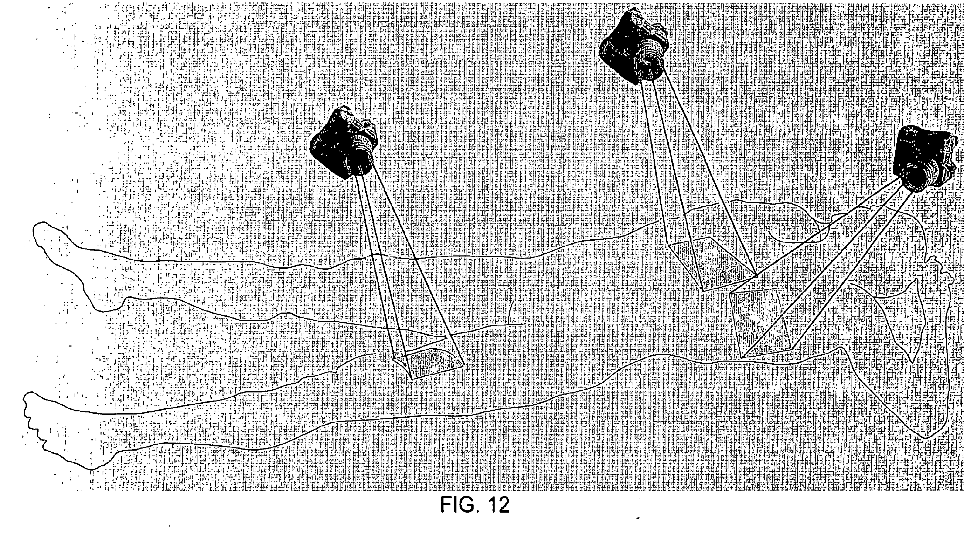

Figure 12 is a three-dimensional view showing three different positions of the image collection means of Fig. 11 with respect to the body of the tested subject;

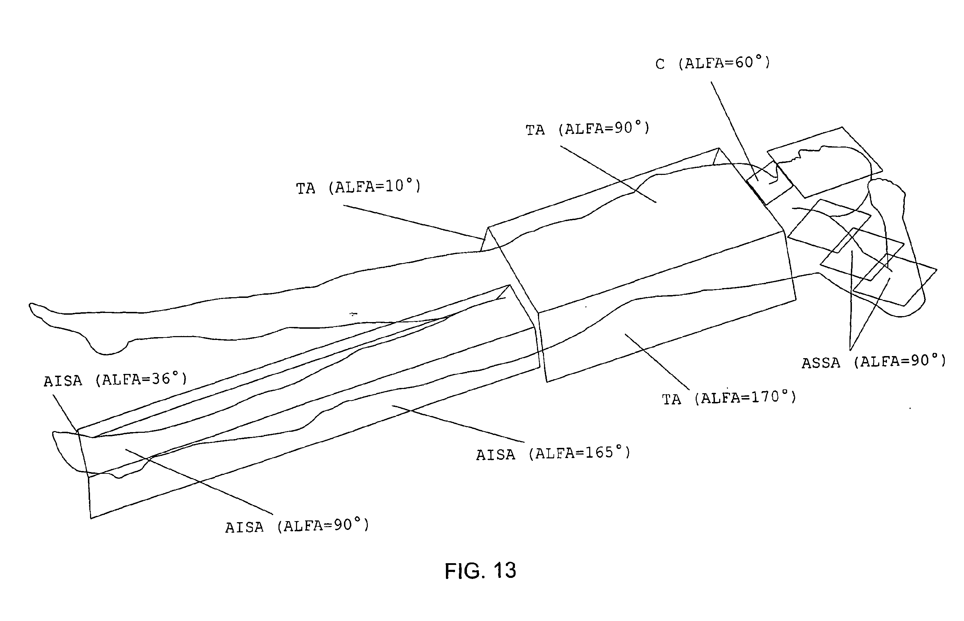

Figure 13 is a view similar to the preceding one showing schematically some reference planes on which the frames of the collected images shown in Figures 6, 9 and 10 lie; and

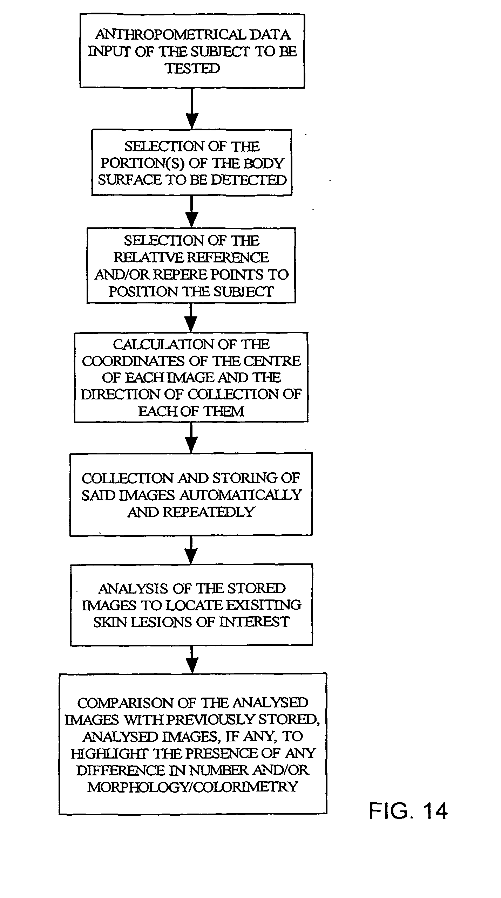

Figure 14 is a block diagram showing the main steps of the method according to the present invention.

From the foregoing it is self-evident that according to the invention the photographic documentation of the skin state includes the whole body and not only some zones selected by the physician during the visit. In order to compare in time the lesions of interest, the collection of the images in subsequent tests is made so that the segmentation of the body provided by the single images is highly reproducible and the little, unavoidable variations of position of the tested subject are minimized until they are compatible with the existing technology.

Moreover, the images to be compared frequently include different "objects": background portions, skin portions and, in some case, underwear portions. Furthermore, the skin portions can include in turn other "objects" besides the pigmentary lesions of interest: solar freckles, angioma, hairs, seborrheic keratosis, etc. Therefore, the automatic comparison of the images is carried out so as to eliminate any source of variability of the image content quite unconnected with the predetermined purposes (modifications of the background, underwear, different orientation of hairs, etc.).

It should be noted that even little modifications of the lighting can influence heavily the colorimetric characteristics of the lesions of interest detected in the images, thus forming a source of false alarms.

The condition necessary to carry out an automatic comparison of the images useful to the predetermined purposes is the suppression or minimization of any variation other than the detected one, i.e. the state of the pigmentary skin lesions of the same subject in time.

According to the present invention, in order to avoid the presence of false variations of the state of the above-mentioned lesions it is provided that:
1. each skin portion of the same subject is detected in subsequent times from a predetermined point of view which is unchanged with respect to the skin surface to be detected;
2. the position and orientation in the space of the camera (defined by Cartesian coordinates on three orthogonal axes X, Y, Z and angular values of the angles of rotation about Y axis (alpha) and Z axis (beta)) can be controlled and connected to the corresponding frame in a corresponding register file;
3. the tested subject is allowed to sit down to a position which is essentially unchanged in any following test;
4. the segmentation of the body into images is made so as to allow a comparison even when modifications of the body of the tested subject take place between subsequent tests.

To this end, the invention provides that the number of images collected for a determined patient is unchanged in the following image collections and that the images have partially overlapped edges to compensate any variation of dimension in the patient.

The distance of the body surface from the image collection means is generally constant and the orientation of such image collection means is predetermined for each image.

In order for the invention to be effective, it is necessary that numeric or morphological/colorimetric modifications of the pigmentary lesions (reset of the false negatives) are shown constantly and that the detection of any modification of other objects included in the compared images (suppression of the false positives) is minimized.

In case the body segmentation performed by the image collection system is absolutely reproducible (two images collected at a distance of time from each other reproducing exactly or almost exactly the same skin portion) as well as the images do not include other structures except for those of interest or other objects which are a source of confusing variations, the comparison is made by matching techniques with image subtraction.

In all of the other cases it is necessary to process the images before their comparison.

Such processing has two main purposes:
- location of objects contained in the image other than skin (underwear, background, etc.) as well as structures that can produce false positives (hairs, spots produced by natural orifices or shadows, tattoos, etc.) with the purpose of ignoring them in the following comparison;
- location of the lesions of interest to be compared. As already mentioned above, the invention is directed to locate automatically the appearance even of only one new nevus or skin lesion of dermatological interest in all of the predefined body portions, for example from a minimum diameter of 2 millimetres on. Moreover, the invention is able to detect, still automatically, a growth of the diameter of one or more of the previously mapped nevi equal for example to 1 millimetre.

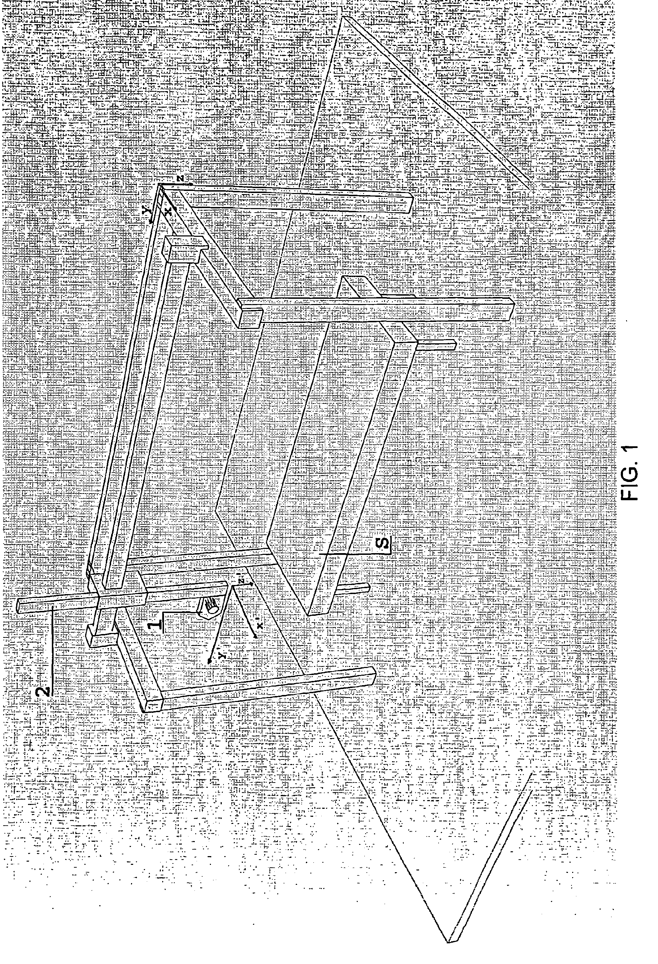

To this end, the method of detection disclosed herein includes the following operating steps:
- subdividing the body surface into quadrants with suitable size (Fig. 1);
- selecting predetermined reference or "repere" points so that the following detection may have repere points able to collimate the body quadrants of the same subject;
- collecting high definition images relative to the above-mentioned quadrants;
- locating, numbering and measuring all of the nevi or skin lesions present in each quadrant;
- highlighting the new skin lesions in each quadrant and/or highlighting the morphological/colorimetric variations in one or more previously located skin lesions.

Such anatomic repere points are, for example, as follows: glabella, labial rima, umbilical jugulum, scapulo-humeral articulation, central point of the median line of cubital cavum, central point of the median line of the fore armpit, insertion of the second interdigital space of the hand, median point of the inguinal fold, lower gluteal fold, spinous eminence of the seventh cervical vertebra.

From a practical point of view, the invention provides an user-oriented apparatus which allows in short time (a few dozens of minutes with respect to many hours necessary in the known methods) people exposed to the risk of melanoma to be checked easily and with a high factor of reliability (for example +10% of false positives, and false negatives near zero).

To do so, the apparatus for carrying out such method includes a robot for driving the image collection means which is remote controlled with precision and reproducibility of its movements.

Moreover, such robot or apparatus for driving the image collection means is able to explore all of the body portions of a subject who is preferably placed in horizontal position on a litter.

The software for processing the collected images is able to measure shape and colour as well as dimension of growth (preferably at least 1 mm), to detect the onset of new skin lesions (for example from 2 mm on), to compare the morphologic and dimensional characteristics of each lesion in the same body portions collected in different times, even in case of little change of position of the tested subject and/or a body modification of the same.

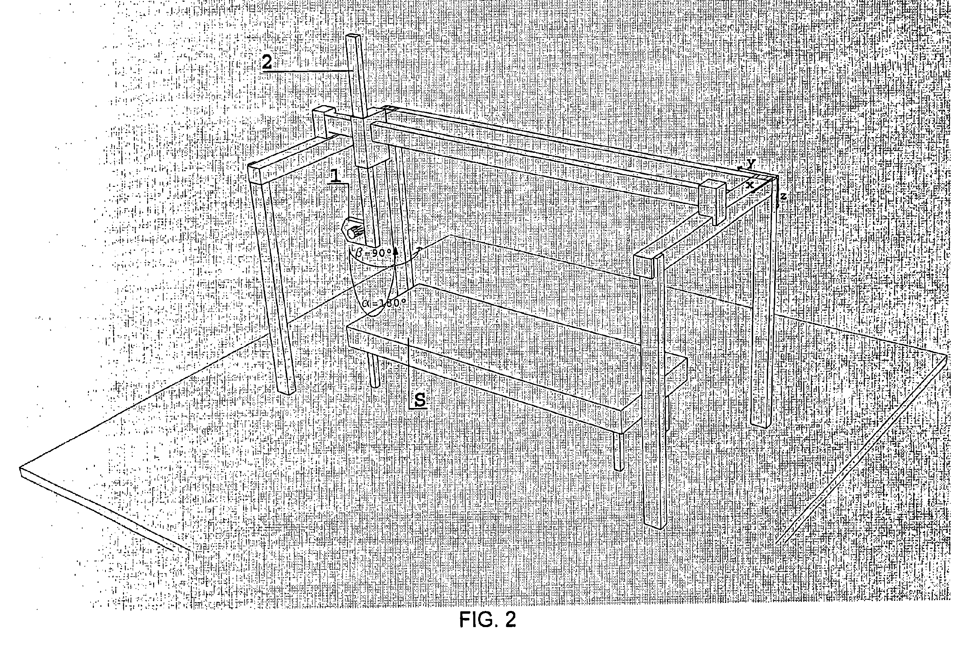

More specifically, the invention provides an apparatus including in combination (Figs. 1-5):
- an application software for processing fine graphic data (skin lesions) provided with algorithms able to provide a discrete set of the detected images (matrices of calculation);
- a data base for the statistic analysis of data of interest;
- a data processing portion for clinic, personal data of the subjects for storing and listing the images of each patient upon his/her visiting (mode of the case history);
- a reference surface S for the tested subject provided with anthropometrical references;
- means for lighting uniformly without shadows the zones of the subject body surface to be detected;
- image collection means 1;
- means 2 for supporting and/or driving under control such image collection means 1 with respect to the patient;
- interface means for controlling the data collection and transmission to suitable storing and/or processing means;
- at least a computer connected to such interface means;
- at least a high definition monitor or video or other display means of the known type;
- means for controlling the correct positioning of the subject.

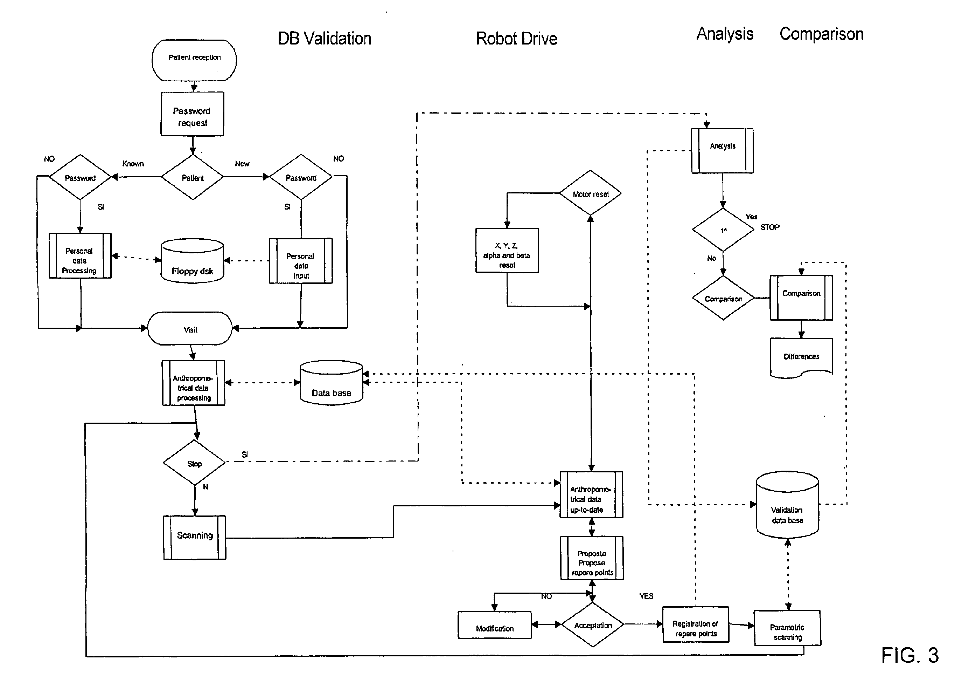

With particular reference to figures 3, 4 and 5, it should be noted that the software disclosed above has a portion relative to the processing of the personal data of the tested subjects which respects of course the rules regarding the privacy and provides the anthropometrical data to define and calculate the coordinates of the selected frames: face, fore trunk, etc., so that they are proposed to the operator for their acceptance or modification before starting the data collection in the automatic mode.

Such portion relative to the privacy of the patient which will not be disclosed into detail makes use obviously of access password.

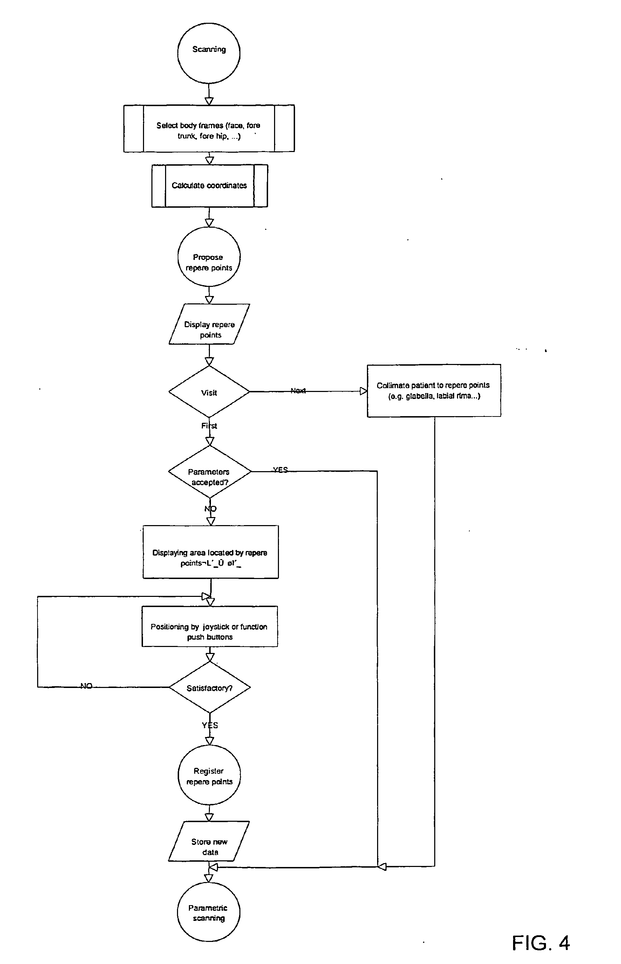

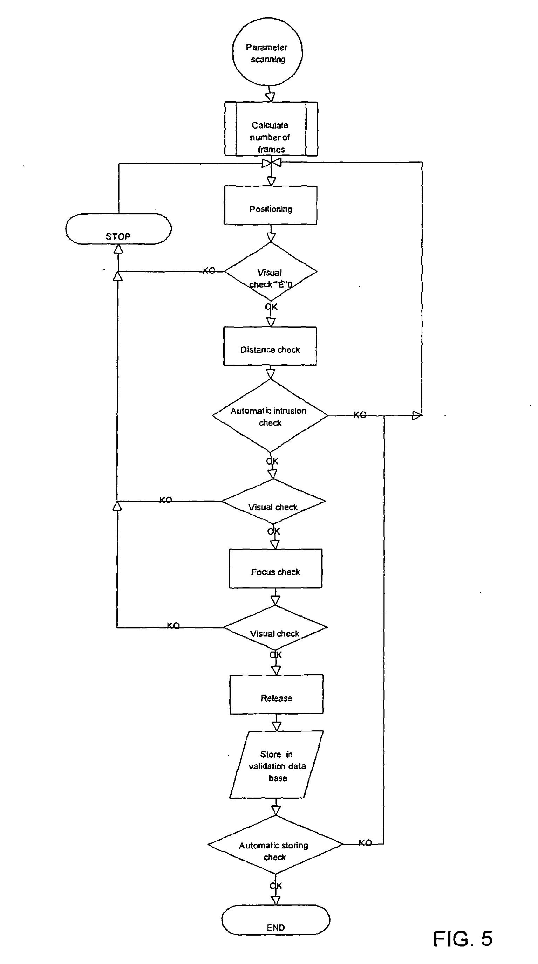

The SCANNING block illustrated in Fig. 4 arranges all of data necessary to the next step of effective PARAMETER SCANNING (Fig. 5).

According to the invention, all of data and images relative to the operations of SCANNING and PARAMETER SCANNING are stored so that they will be available for the next processing and/or comparison with data and images collected previously or subsequently.

The block ANALYSIS (Fig. 3) is arranged for the "cleaning" of the images collected from the objects not pertaining to the scanning performed, such as underwear, hairs, tattoos, other unconnected objects which would hamper a correct comparison able to locate all differences in the skin of the tested subject with respect to the preceding situation. Such cleaning operation is performed preferably by calculation routines of the known type in the field of graphic image processing.

It should also be noted that the portion of the software relative to the personal data processing of the subjects in case history mode is not necessary in itself for accomplishing the invention, however, it is indispensable to protect the privacy of the tested subjects. In fact it would be enough for the purposes of the invention to identify each subject by an univocal code (such as his/her fiscal code) to which the data base associates all data and images relative to the tested subject.

In the disclosed embodiment the block ANALYSIS performs the following operations:
- recognizing not human pixels;
- ablating piliferous appendages by parametrization software;
- constructing grey levels referred to the weight of blue;
- constructing the background (smoothing);
- constructing levels of identification of the pigmented areas (spot objects);
- calculating mathematically the evidence threshold;
- recognizing the pigmented areas (spot objects);
- characterizing the pigmented areas in terms of their specific qualities (spot objects);
- differentiating the pigmented areas (spot objects) of the background noise (hair, underwear, tattoos, orifices, etc. objects).

Still in the disclosed embodiment, the block COMPARISON performs the following operations:
- collimating frames (algorithm 1);
- rotating/translating in scale;
- calculating the known connections;
- translating the pigmented areas (spot objects) to an assigned range to minimize the discards;
- calculating the differences (minus and/or plus dimensional - variation of the inner colour);
- optimising: if the number of erroneous connections is greater than three or if the secondary translation of the pigmented areas (spot objects) is greater than an assigned level (for example 100 pixels), a second collimation method (algorithm 2) is performed.

It should be noted that the anthropometrical references located in the reference surface for the subject (in the example shown it is preferably a litter) are necessary to locate the repere points which are significant for different somatic types as well as for the same patient subjected to several next detections so that defined, significant body areas that can be overlapped are obtained (collimation of the taken images).

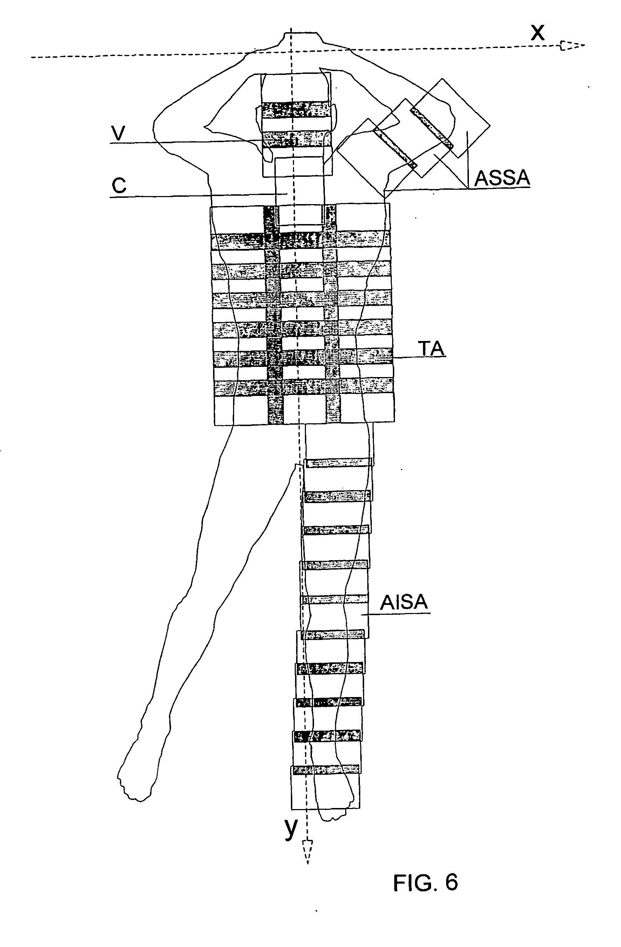

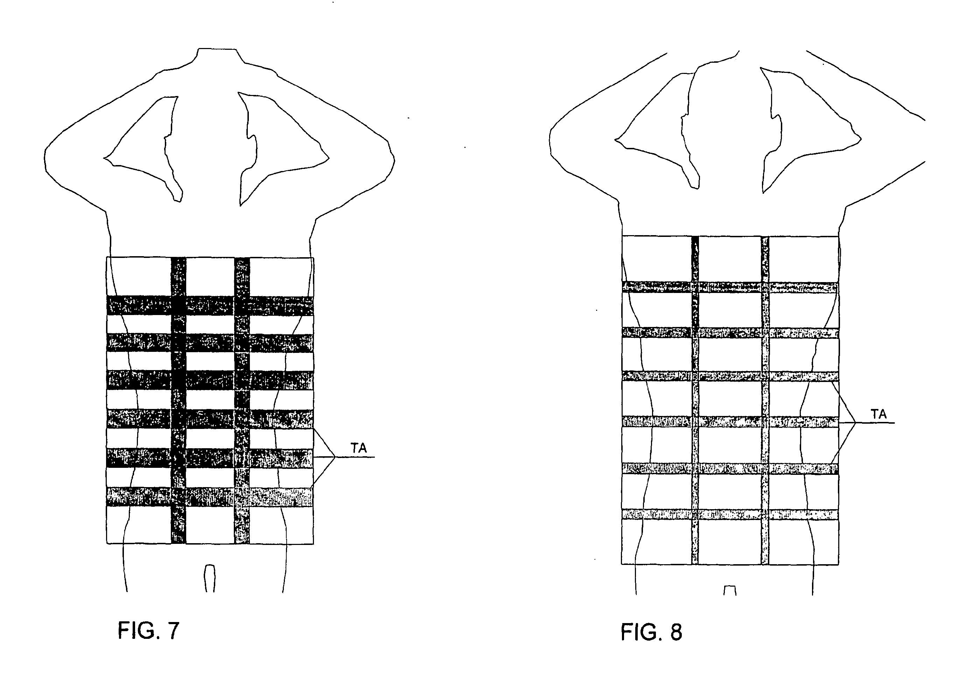

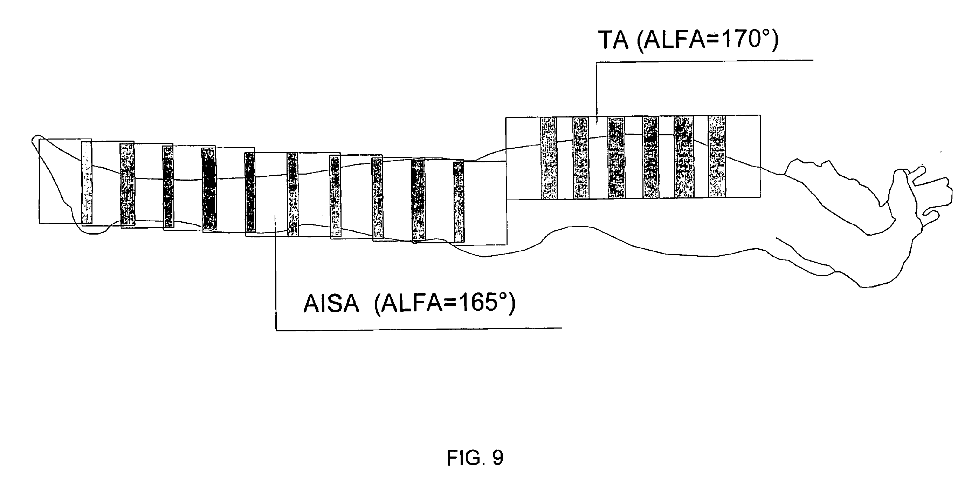

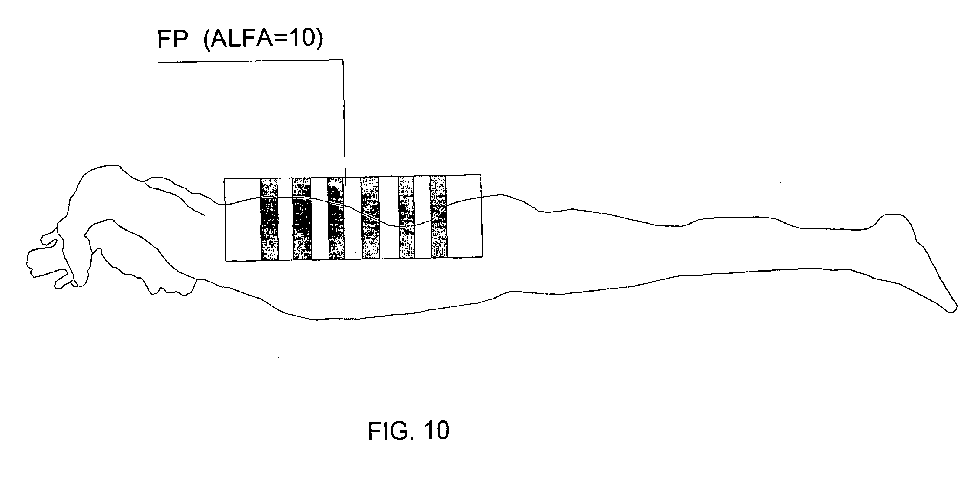

The overlap of the image edges adjacent to one another varies preferably from a maximum equal to half the height and half the width of the image to a minimum that can be zero (images with coincident edges). Figures 6-10 show by way of example some possible divisions into quadrants of the body surface of a subject to be tested.

The following table 1 indicates the meaning of the symbols used in last-mentioned figures.

Further tables listing the coordinates and angles of each image with reference to the symbols of the above-mentioned table 1 are shown to better explain the shifts of the drive apparatus during the image collection.

As can be seen, one or more sets of images, each of them being characterized by a precise position (coordinates x, y, z) and a precise orientation of the collecting means (angles alpha and beta), are collected, the distance from the body surface being hold to an almost constant value by suitable proximity detection means. Thus, the focal distance and the area covered thereby is kept for each image unchanged.

It should be appreciated that these values are of course valid for the embodiment illustrated herein by way of example only, however, they can vary as a function of the construction executed.

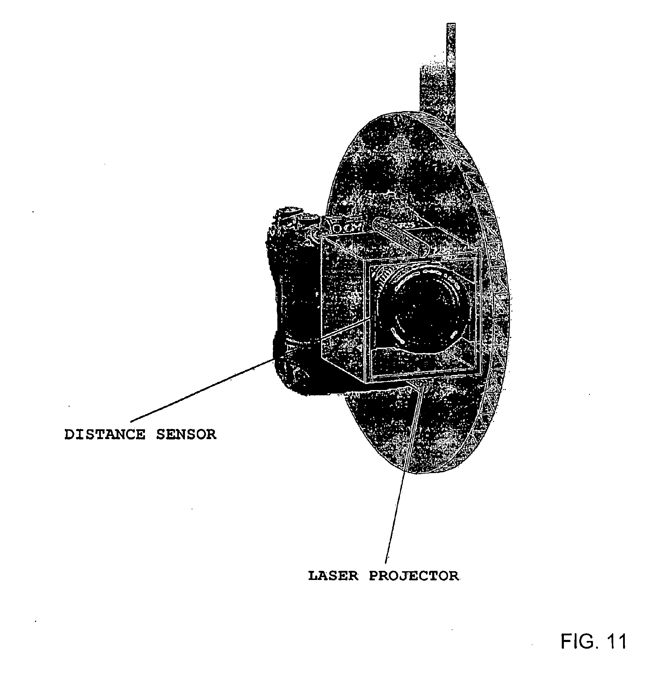

In the preferred embodiment disclosed, such proximity detection means consists of radiofrequency (ultrasounds) sensors disposed at the right and left sides of the image collection means (Fig. 11). According to the present invention, if the distance between such image collection means and the body surface of the subject to be checked is lower than a predetermined value, the apparatus is immediately blocked and the intervention of an operator is requested to check the causes of the failure and to restart the system.

**Table 1**

| | | | |
|---|---|---|---|
| Fore right lower extremity | AIDA | Inner left extremity | BSI |
| Rear right lower extremity | AIDP | Neck | C |
| Fore left lower extremity | AISA | Foot back | DP |
| Rear left lower extremity | AISP | Fore hips | FA |
| Fore right upper extremity | ASDA | Rear hips | FP |
| Inner right upper extremity | ASDI | Absolute repere | RA |
| Rear right upper extremity | ASDP | Rear right shoulder | SDP |
| Fore left upper extremity | ASSA | Fore left shoulder | SSA |
| Inner left upper extremity | ASSI | Fore trunk | TA |
| Rear left upper extremity | ASSP | Rear trunk | TP |
| Inner right extremity | BDI | Face | V |

**Table BSI**

| **File Name** | **X** | **Y** | **Z** | **Alpha** | **Beta** | **Dlf** | **Drt** |
|---|---|---|---|---|---|---|---|
| Image001.jpg | 901.95 | 387.54 | 228.23 | 90.00 | 0.00 | 51.72 | 53.84 |
| Image002.jpg | 970.42 | 308.86 | 244.02 | 90.00 | 0.00 | 54.02 | 51.84 |
| Image003.jpg | 1038.89 | 230.03 | 245.23 | 90.00 | 0.00 | 52.94 | 62.42 |

**Table AIDP**

| **File Name** | **X** | **Y** | **Z** | **Alpha** | **Beta** | **Dlf** | **Drt** |
|---|---|---|---|---|---|---|---|
| Image001.jpg | 824.32 | 1035.74 | 178.50 | 90.00 | 0.00 | 55.70 | 50.70 |
| Image002.jpg | 822.07 | 1129.58 | 210.87 | 90.00 | 0.00 | 53.81 | 52.41 |
| Image003.jpg | 819.82 | 1223.57 | 228.41 | 90.00 | 0.00 | 53.42 | 52.47 |
| Image004.jpg | 817.57 | 1317.42 | 234.17 | 90.00 | 0.00 | 51.61 | 54.40 |
| Image005.jpg | 815.17 | 1411.26 | 215.68 | 90.00 | 0.00 | 51.94 | 54.34 |
| Image006.jpg | 813.06 | 1505.26 | 188.53 | 90.00 | 0.00 | 52.20 | 53.61 |
| Image007.jpg | 810.66 | 1599.10 | 188.05 | 90.00 | 0.00 | 71.22 | 52.96 |
| Image008.jpg | 808.41 | 1692.94 | 164.44 | 90.00 | 0.00 | 66.00 | 52.90 |
| Image009.jpg | 806.16 | 1786.94 | 102.40 | 90.00 | 0.00 | 65.90 | 52.71 |

**Table C**

| **File Name** | **X** | **Y** | **Z** | **Alpha** | **Beta** | **Dlf** | **Drt I** |
|---|---|---|---|---|---|---|---|
| Image001.jpg | 733.93 | 600.90 | 239.70 | 60.00 | 90.00 | 56.28 | 68.58 |

**Table AI DA:**

| **File Name** | **X** | **Y** | **Z** | **Alpha** | **Beta** | **Dlf** | **Drt** |
|---|---|---|---|---|---|---|---|
| image001.jpg | 99.55 | 947.00 | 602.40 | 15.00 | 0.00 | 53.83 | 51.71 |
| image002.jpg | 101.95 | 1052.25 | 602.88 | 15.00 | 0.00 | 53.39 | 52.10 |
| image003.jpg | 127.63 | 1157.51 | 609.61 | 15.00 | 0.00 | 54.79 | 51.18 |
| image004.jpg | 135.29 | 1262.76 | 611.53 | 15.00 | 0.00 | 53.26 | 52.18 |
| image005.jpg | 159.16 | 1368.02 | 617.60 | 15.00 | 0.00 | 53.26 | 52.71 |
| image006.jpg | 164.71 | 1473.27 | 618.98 | 15.00 | 0.00 | 52.53 | 53.55 |
| image007.jpg | 177.18 | 1578.53 | 622.04 | 15.00 | 0.00 | 52.15 | 53.64 |
| image008.jpg | 164.26 | 1683.78 | 618.56 | 15.00 | 0.00 | 52.38 | 61.68 |
| image009.jpg | 199.55 | 1789.04 | 627.75 | 15.00 | 0.00 | 51.83 | 54.13 |
| image010.jpg | 678.83 | 1002.85 | 123.00 | 90.00 | 0.00 | 58.63 | 52.96 |
| image011.jpg | 679.43 | 1101.05 | 192.31 | 90.00 | 0.00 | 54.21 | 51.89 |
| image012.jpg | 680.03 | 1199.40 | 203.72 | 90.00 | 0.00 | 52.45 | 53.49 |
| image013.jpg | 680.78 | 1297.75 | 160.54 | 90.00 | 0.00 | 52.24 | 60.81 |
| image014.jpg | 681.38 | 1395.95 | 218.20 | 90.00 | 0.00 | 52.72 | 54.74 |
| image015.jpg | 682.13 | 1494.14 | 259.64 | 90.00 | 0.00 | 52.84 | 53.21 |
| image016.jpg | 682.73 | 1592.49 | 245.35 | 90.00 | 0.00 | 51.56 | 54.37 |
| image017.jpg | 683.33 | 1690.69 | 244.92 | 90.00 | 0.00 | 53.26 | 58.42 |
| image018.jpg | 683.93 | 1789.04 | 200.30 | 90.00 | 0.00 | 46.46 | 65.32 |
| Image019.jpg | 1222.22 | 1086.94 | 365.11 | 144.00 | 0.00 | 63.86 | 53.21 |
| Image020.jpg | 1136.19 | 1174.77 | 427.99 | 144.00 | 0.00 | 55.28 | 50.48 |
| Image021.jpg | 1119.37 | 1262.46 | 440.78 | 144.00 | 0.00 | 53.26 | 52.47 |
| Image022.jpg | 1111.11 | 1350.15 | 447.15 | 144.00 | 0.00 | 52.53 | 53.07 |
| Image023.jpg | 1094.89 | 1437.99 | 459.52 | 144.00 | 0.00 | 53.70 | 52.12 |
| Image024.jpg | 1094.44 | 1525.83 | 460.30 | 144.00 | 0.00 | 52.91 | 52.79 |
| Image025.jpg | 1104.05 | 1613.51 | 453.63 | 144.00 | 0.00 | 54.07 | 52.20 |
| Image026.jpg | 1102.85 | 1701.20 | 455.02 | 144.00 | 0.00 | 51.43 | 53.99 |
| Image027.jpg | 1112.31 | 1789.04 | 448.77 | 144.00 | 0.00 | 52.36 | 58.35 |

**Table AISA**

| **File Name** | **X** | **Y** | **Z** | **Alpha** | **Beta** | **Dlf** | **Drt** |
|---|---|---|---|---|---|---|---|
| Image001.jpg | 263.06 | 1086.94 | 367.21 | 36.00 | 0.00 | 62.40 | 52.88 |
| Image002.jpg | 356.46 | 1174.77 | 439.70 | 36.00 | 0.00 | 53.83 | 51.91 |
| Image003.jpg | 366.37 | 1262.46 | 451.29 | 36.00 | 0.00 | 53.14 | 52.88 |
| image004.jpg | 363.21 | 1350.15 | 453.69 | 36.00 | 0.00 | 53.52 | 52.63 |
| image005.jpg | 374.47 | 1437.99 | 466.19 | 36.00 | 0.00 | 52.79 | 52.55 |
| image006.jpg | 382.28 | 1525.68 | 476.58 | 36.00 | 0.00 | 52.74 | 53.41 |
| image007.jpg | 408.56 | 1613.51 | 500.12 | 36.00 | 0.00 | 56.41 | 50.74 |
| image008.jpg | 390.54 | 1701.20 | 491.47 | 36.00 | 0.00 | 55.11 | 52.20 |
| image009.jpg | 374.47 | 1789.04 | 484.44 | 36.00 | 0.00 | 60.04 | 52.90 |
| image010.jpg | 833.93 | 1023.72 | 174.65 | 90.00 | 0.00 | 53.73 | 52.10 |
| image011.jpg | 827.63 | 1119.37 | 181.44 | 90.00 | 0.00 | 53.68 | 52.23 |
| image012.jpg | 821.47 | 1215.02 | 181.62 | 90.00 | 0.00 | 53.55 | 52.55 |
| image013.jpg | 815.17 | 1310.66 | 181.14 | 90.00 | 0.00 | 50.80 | 55.05 |
| image014.jpg | 809.01 | 1406.46 | 225.59 | 90.00 | 0.00 | 53.91 | 52.28 |
| image015.jpg | 802.70 | 1501.95 | 206.37 | 90.00 | 0.00 | 54.91 | 50.86 |
| image016.jpg | 796.55 | 1597.75 | 255.08 | 90.00 | 0.00 | 62.53 | 53.01 |
| image017.jpg | 790.24 | 1693.24 | 240.18 | 90.00 | 0.00 | 63.45 | 52.96 |
| image018.jpg | 783.93 | 1789.04 | 277.48 | 90.00 | 0.00 | 55.16 | 55.59 |
| image019.jpg | 1402.70 | 978.23 | 612.73 | 165.00 | 0.00 | 54.43 | 51.16 |
| image020.jpg | 1383.33 | 1079.58 | 616.28 | 165.00 | 0.00 | 52.65 | 52.58 |
| image021.jpg | 1367.87 | 1180.93 | 618.74 | 165.00 | 0.00 | 53.11 | 52.31 |
| image022.jpg | 1345.95 | 1282.28 | 623.00 | 165.00 | 0.00 | 53.65 | 51.99 |
| image023.jpg | 1340.69 | 1383.63 | 622.76 | 165.00 | 0.00 | 53.19 | 52.63 |
| image024.jpg | 1354.35 | 1484.98 | 617.48 | 165.00 | 0.00 | 50.39 | 54.95 |
| image025.jpg | 1361.26 | 1586.34 | 613.87 | 165.00 | 0.00 | 89.00 | 52.52 |
| image026.jpg | 1332.13 | 1687.69 | 620.06 | 165.00 | 0.00 | 56.95 | 50.39 |
| image027.jpg | 1322.67 | 1789.04 | 620.90 | 165.00 | 0.00 | 53.26 | 51.46 |

**Table AISP**

| **File Name** | **X** | **Y** | **Z** | **Alpha** | **Beta** | **Dlf** | **Drt** |
|---|---|---|---|---|---|---|---|
| Image001.jpg | 633.93 | 1013.51 | 123.90 | 90.00 | 0.00 | 59.47 | 53.10 |
| Image002.jpg | 633.93 | 1110.21 | 221.92 | 90.00 | 0.00 | 51.63 | 49.27 |
| Image003.jpg | 633.93 | 1206.91 | 212.13 | 90.00 | 0.00 | 52.89 | 53.24 |
| Image004.jpg | 633.93 | 1303.60 | 227.39 | 90.00 | 0.00 | 52.74 | 53.61 |
| Image005.jpg | 633.93 | 1400.30 | 220.12 | 90.00 | 0.00 | 52.20 | 54.13 |
| Image006.jpg | 633.93 | 1496.85 | 222.58 | 90.00 | 0.00 | 51.67 | 54.16 |
| Image007.jpg | 633.93 | 1593.54 | 222.28 | 90.00 | 0.00 | 52.43 | 53.93 |
| Image008.jpg | 633.93 | 1690.24 | 177.84 | 90.00 | 0.00 | 52.84 | 67.32 |
| Image009.jpg | 633.93 | 1786.94 | 232.31 | 90.00 | 0.00 | 52.01 | 55.30 |

**Table ASDA**

| **File Name** | **X** | **Y** | **Z** | **Alpha** | **Beta** | **Dlf** | **Drt** |
|---|---|---|---|---|---|---|---|
| Image001.jpg | 20.87 | 449.25 | 637.06 | 10.00 | 0.00 | 52.84 | 60.08 |
| Image002.jpg | 25.98 | 541.89 | 638.50 | 10.00 | 0.00 | 52.67 | 53.32 |
| image003.jpg | 33.48 | 634.53 | 640.18 | 10.00 | 0.00 | 52.38 | 53.58 |
| image004.jpg | 28.08 | 727.03 | 639.76 | 10.00 | 0.00 | 53.14 | 52.77 |
| image005.jpg | 19.67 | 819.67 | 638.62 | 10.00 | 0.00 | 54.26 | 51.73 |
| image006.jpg | 15.62 | 912.16 | 638.44 | 10.00 | 0.00 | 53.01 | 52.41 |
| image007.jpg | 4.80 | 1004.95 | 636.82 | 10.00 | 0.00 | 52.79 | 51.58 |
| image008.jpg | 44.29 | 1097.45 | 644.26 | 10.00 | 0.00 | 55.19 | 50.77 |
| image009.jpg | 573.57 | 449.25 | 176.10 | 90.00 | 0.00 | 53.06 | 65.27 |
| image010.jpg | 571.17 | 541.89 | 178.50 | 90.00 | 0.00 | 54.40 | 51.63 |
| image011.jpg | 568.62 | 634.53 | 200.00 | 90.00 | 0.00 | 53.97 | 52.15 |
| image012.jpg | 566.07 | 727.03 | 196.40 | 90.00 | 0.00 | 53.65 | 52.41 |
| image013.jpg | 563.51 | 819.67 | 199.46 | 90.00 | 0.00 | 51.13 | 55.08 |
| image014.jpg | 560.96 | 912.16 | 181.14 | 90.00 | 0.00 | 50.84 | 55.02 |
| image015.jpg | 558.56 | 1004.95 | 160.36 | 90.00 | 0.00 | 53.01 | 52.47 |
| image016.jpg | 556.01 | 1097.45 | 156.04 | 90.00 | 0.00 | 67.26 | 52.88 |

**Table ASDI**

| **File Name** | **X** | **Y** | **Z** | **Alpha** | **Beta** | **Dlf** | **Drt** |
|---|---|---|---|---|---|---|---|
| Image001.jpg | 433.18 | 637.69 | 230.75 | 90.00 | 0.00 | 51.63 | 54.25 |
| Image002.jpg | 360.96 | 737.54 | 202.76 | 90.00 | 0.00 | 54.91 | 50.96 |
| Image003.jpg | 288.74 | 837.24 | 227.39 | 90.00 | 0.00 | 52.91 | 61.81 |
| Image004.jpg | 216.52 | 937.09 | 210.27 | 90.00 | 0.00 | 56.31 | 52.33 |

**Table ASDP**

| **File Name** | **X** | **Y** | **Z** | **Alpha** | **Beta** | **Dlf** | **Drt** |
|---|---|---|---|---|---|---|---|
| Image001.jpg | 935.44 | 410.36 | 190.45 | 90.00 | 0.00 | 51.15 | 57.59 |
| Image002.jpg | 938.74 | 508.86 | 202.88 | 90.00 | 0.00 | 53.11 | 58.17 |
| Image003.jpg | 942.19 | 607.51 | 224.86 | 90.00 | 0.00 | 50.96 | 55.36 |
| Image004.jpg | 945.65 | 706.16 | 234.65 | 90.00 | 0.00 | 54.59 | 51.56 |
| Image005.jpg | 949.10 | 804.65 | 258.32 | 90.00 | 0.00 | 55.64 | 51.21 |
| Image006.jpg | 952.55 | 903.15 | 238.74 | 90.00 | 0.00 | 51.45 | 54.34 |
| Image007.jpg | 955.86 | 1001.80 | 266.13 | 90.00 | 0.00 | 53.09 | 52.82 |

**Table ASSP**

| **File Name** | **X** | **Y** | **Z** | **Alpha** | **Beta** | **Dlf** | **Drt** |
|---|---|---|---|---|---|---|---|
| image001.jpg | 537.09 | 441.14 | 171.47 | 90.00 | 0.00 | 52.77 | 75.39 |
| image002.jpg | 535.44 | 541.44 | 206.19 | 90.00 | 0.00 | 51.85 | 54.34 |
| image003.jpg | 533.63 | 641.74 | 226.07 | 90.00 | 0.00 | 53.57 | 52.63 |
| image004.jpg | 532.13 | 742.04 | 243.06 | 90.00 | 0.00 | 54.37 | 51.46 |
| image005.jpg | 530.33 | 842.34 | 298.86 | 90.00 | 0.00 | 55.31 | 50.86 |
| image006.jpg | 528.68 | 942.79 | 263.66 | 90.00 | 0.00 | 53.37 | 52.18 |
| image007.jpg | 527.03 | 1043.09 | 322.88 | 90.00 | 0.00 | 53.94 | 51.89 |

**Table ASSA**

| **File Name** | **X** | **Y** | **Z** | **Alpha** | **Beta** | **Dlf** | **Drt** |
|---|---|---|---|---|---|---|---|
| Image001.jpg | 896.85 | 427.03 | 166.19 | 90.00 | 0.00 | 52.91 | 64.51 |
| Image002.jpg | 906.46 | 515.77 | 139.22 | 90.00 | 0.00 | 52.84 | 59.80 |
| Image003.jpg | 916.07 | 604.35 | 196.88 | 90.00 | 0.00 | 52.13 | 53.78 |
| Image004.jpg | 925.53 | 692.94 | 168.71 | 90.00 | 0.00 | 52.45 | 59.25 |
| image005.jpg | 935.14 | 781.53 | 221.32 | 90.00 | 0.00 | 53.26 | 50.96 |
| image006.jpg | 944.74 | 870.27 | 209.55 | 90.00 | 0.00 | 52.48 | 53.38 |
| image007.jpg | 954.35 | 958.86 | 207.93 | 90.00 | 0.00 | 52.48 | 53.46 |
| image008.jpg | 963.96 | 1047.45 | 236.10 | 90.00 | 0.00 | 52.84 | 53.21 |
| image009.jpg | 1439.34 | 427.03 | 620.84 | 170.00 | 0.00 | 52.69 | 75.00 |
| image010.jpg | 1427.18 | 515.62 | 624.74 | 170.00 | 0.00 | 51.97 | 53.27 |
| image011.jpg | 1456.61 | 604.35 | 621.14 | 170.00 | 0.00 | 66.44 | 52.77 |
| image012.jpg | 1445.20 | 692.94 | 624.86 | 170.00 | 0.00 | 54.02 | 51.94 |
| image013.jpg | 1440.69 | 781.53 | 627.27 | 170.00 | 0.00 | 64.36 | 53.04 |
| image014.jpg | 1476.43 | 870.27 | 622.70 | 170.00 | 0.00 | 61.72 | 52.77 |
| image015.jpg | 1458.11 | 958.86 | 627.63 | 170.00 | 0.00 | 53.34 | 51.76 |
| image016.jpg | 1432.88 | 1047.45 | 633.87 | 170.00 | 0.00 | 54.02 | 51.38 |

**Table ASSI**

| **File Name** | **X** | **Y** | **Z** | **Alpha** | **Beta** | **Dlf** | **Drt** |
|---|---|---|---|---|---|---|---|
| Image001.jpg | 1015.32 | 671.92 | 209.79 | 90.00 | 0.00 | 53.73 | 52.44 |
| Image002.jpg | 1077.78 | 777.18 | 233.39 | 90.00 | 0.00 | 62.70 | 53.32 |
| Image003.jpg | 1140.24 | 882.43 | 265.77 | 90.00 | 0.00 | 58.48 | 53.24 |
| Image004.jpg | 1202.70 | 987.69 | 230.15 | 90.00 | 0.00 | 59.58 | 53.04 |

**Table BDI**

| **File Name** | **X** | **Y** | **Z** | **Alpha** | **Beta** | **Dlf** | **Drt** |
|---|---|---|---|---|---|---|---|
| image001.jpg | 562.31 | 397.75 | 209.67 | 90.00 | 0.00 | 53.06 | 59.84 |
| image002.jpg | 499.40 | 321.17 | 257.12 | 90.00 | 0.00 | 51.30 | 54.71 |
| image003.jpg | 436.49 | 244.74 | 211.53 | 90.00 | 0.00 | 52.53 | 65.51 |

**Table DP**

| **File Name** | **X** | **Y** | **Z** | **Alpha** | **Beta** | **Dlf** | **Drt** |
|---|---|---|---|---|---|---|---|
| Image001.jpg | 669.37 | 1406.61 | 321.44 | 140.00 | 90.00 | 61.60 | 88.16 |
| Image002.jpg | 817.87 | 1406.61 | 321.44 | 140.00 | 90.00 | 70.30 | 66.35 |

**Table RA**

| **File Name** | **X** | **Y** | **Z** | **Alpha** | **Beta** | **Dlf** | **Drt** |
|---|---|---|---|---|---|---|---|
| Image001.jpg | 733.93 | 1038.29 | 80.00 | 90.00 | 0.00 | 64.36 | 56.44 |

**Table SDP**

| **File Name** | **X** | **Y** | **Z** | **Alpha** | **Beta** | **Dlf** | **Drt** |
|---|---|---|---|---|---|---|---|
| Image001.jpg | 1131.53 | 21.77 | 604.02 | 165.00 | 50.00 | 49.21 | 54.77 |

**Table FA**

| **File Name** | **X** | **Y** | **Z** | **Alpha** | **Beta** | **Dlf** | **Drt** |
|---|---|---|---|---|---|---|---|
| Image001.jpg | 120.57 | 438.74 | 586.67 | 10.00 | 0.00 | 52.89 | 66.20 |
| Image002.jpg | 145.50 | 525.38 | 590.87 | 10.00 | 0.00 | 50.73 | 55.33 |
| Image003.jpg | 127.93 | 612.01 | 587.93 | 10.00 | 0.00 | 51.09 | 54.74 |
| Image004.jpg | 123.72 | 698.65 | 587.27 | 10.00 | 0.00 | 52.91 | 52.41 |
| Image005.jpg | 128.53 | 785.29 | 588.05 | 10.00 | 0.00 | 53.86 | 52.10 |
| Image006.jpg | 124.17 | 871.92 | 587.27 | 10.00 | 0.00 | 50.43 | 56.21 |
| Image007.jpg | 1368.92 | 438.74 | 597.72 | 170.00 | 0.00 | 52.01 | 61.73 |
| Image008.jpg | 1362.46 | 525.38 | 598.92 | 170.00 | 0.00 | 53.31 | 52.12 |
| Image009.jpg | 1364.11 | 612.01 | 598.62 | 170.00 | 0.00 | 52.06 | 53.18 |
| Image010.jpg | 1369.37 | 698.65 | 597.60 | 170.00 | 0.00 | 52.03 | 53.10 |
| Image011.jpg | 1361.11 | 785.29 | 599.10 | 170.00 | 0.00 | 53.44 | 52.10 |
| Image012.jpg | 1383.78 | 871.92 | 595.08 | 170.00 | 0.00 | 51.56 | 53.27 |

**Table FP**

| **File** Name | **X** | **Y** | **Z** | **Alpha** | **Beta** | **Dlf** | **Drt** |
|---|---|---|---|---|---|---|---|
| image001.jpg | 70.27 | 367.72 | 577.72 | 10.00 | 0.00 | 52.60 | 68.80 |
| image002.jpg | 108.26 | 460.96 | 584.44 | 10.00 | 0.00 | 50.49 | 55.81 |
| image003.jpg | 103.15 | 554.20 | 583.48 | 10.00 | 0.00 | 53.89 | 51.84 |
| image004.jpg | 124.77 | 647.45 | 587.33 | 10.00 | 0.00 | 53.26 | 52.63 |
| image005.jpg | 123.27 | 740.69 | 587.15 | 10.00 | 0.00 | 51.56 | 54.89 |
| image006.jpg | 85.29 | 833.93 | 580.42 | 10.00 | 0.00 | 52.36 | 59.45 |
| image007.jpg | 1437.54 | 367.72 | 599.52 | 170.00 | 0.00 | 52.72 | 66.61 |
| image008.jpg | 1470.42 | 460.96 | 593.81 | 170.00 | 0.00 | 59.06 | 52.79 |
| image009.jpg | 1405.86 | 554.20 | 605.11 | 170.00 | 0.00 | 53.57 | 51.43 |
| image010.jpg | 1378.68 | 647.45 | 609.91 | 170.00 | 0.00 | 53.34 | 51.76 |
| image011.jpg | 1383.78 | 740.69 | 609.01 | 170.00 | 0.00 | 52.69 | 52.39 |
| image012.jpg | 1379.13 | 833.93 | 609.85 | 170.00 | 0.00 | 52.74 | 52.18 |

**Table SSA**

| **File Name** | **X** | **Y** | **Z** | **Alpha** | **Beta** | **Dlf** | **Drt** |
|---|---|---|---|---|---|---|---|
| Image001.jpg | 1131.53 | 21.77 | 604.02 | 165.00 | 50.00 | 48.50 | 53.38 |

**Table V**

| **File Name** | **X** | **Y** | **Z** | **Alpha** | **Beta** | **Dlf** | **Drt** |
|---|---|---|---|---|---|---|---|
| Image001.jpg | 177.48 | 207.06 | 577.60 | 10.00 | 0.00 | 53.49 | 53.04 |
| Image002.jpg | 100.90 | 322.37 | 564.20 | 10.00 | 0.00 | 63.41 | 52.77 |
| Image003.jpg | 733.93 | 207.06 | 123.36 | 90.00 | 0.00 | 52.77 | 61.47 |
| Image004.jpg | 733.93 | 322.37 | 126.07 | 90.00 | 0.00 | 53.99 | 51.99 |
| Image005.jpg | 1273.42 | 207.06 | 580.54 | 170.00 | 0.00 | 53.16 | 88.16 |
| Image006.jpg | 1256.61 | 322.37 | 583.54 | 170.00 | 0.00 | 52.48 | 53.75 |

**Table TA**

| **File Name** | **X** | **Y** | **Z** | **Alpha** | **Beta** | **Dlf** | **Drt** |
|---|---|---|---|---|---|---|---|
| Image001.jpg | 633.93 | 438.74 | 155.20 | 90.00 | 0.00 | 53.16 | 75.45 |
| Image002.jpg | 633.93 | 525.38 | 101.68 | 90.00 | 0.00 | 52.94 | 60.12 |
| Image003.jpg | 633.93 | 612.01 | 112.49 | 90.00 | 0.00 | 52.10 | 54.04 |
| Image004.jpg | 633.93 | 698.65 | 113.99 | 90.00 | 0.00 | 56.00 | 50.77 |
| Image005.jpg | 633.93 | 785.29 | 141.14 | 90.00 | 0.00 | 55.22 | 50.79 |
| Image006.jpg | 633.93 | 871.92 | 154.53 | 90.00 | 0.00 | 53.68 | 51.63 |
| Image007.jpg | 733.93 | 438.74 | 128.65 | 90.00 | 0.00 | 53.68 | 51.94 |
| Image008.jpg | 733.93 | 525.38 | 126.43 | 90.00 | 0.00 | 52.57 | 53.41 |
| Image009.jpg | 733.93 | 612.01 | 123.30 | 90.00 | 0.00 | 53.04 | 53.10 |
| Image010.jpg | 733.93 | 698.65 | 105.59 | 90.00 | 0.00 | 54.85 | 51.51 |
| Image011.jpg | 733.93 | 785.29 | 111.89 | 90.00 | 0.00 | 54.62 | 51.46 |
| Image012.jpg | 733.93 | 871.92 | 128.29 | 90.00 | 0.00 | 54.79 | 51.63 |
| Image013.jpg | 833.93 | 438.74 | 167.81 | 90.00 | 0.00 | 51.63 | 54.40 |
| Image014.jpg | 833.93 | 525.38 | 157.12 | 90.00 | 0.00 | 51.24 | 54.83 |
| Image015.jpg | 833.93 | 612.01 | 107.81 | 90.00 | 0.00 | 53.06 | 62.37 |
| Image016.jpg | 833.93 | 698.65 | 155.50 | 90.00 | 0.00 | 53.37 | 52.60 |
| Image017.jpg | 833.93 | 785.29 | 153.99 | 90.00 | 0.00 | 54.71 | 51.13 |
| Image018.jpg | 833.93 | 871.92 | 167.39 | 90.00 | 0.00 | 52.29 | 53.78 |

**Table TP**

| **File Name** | **X** | **Y** | **Z** | **Alpha** | **Beta** | **Dlf** | **Drt** |
|---|---|---|---|---|---|---|---|
| image001.jpg | 633.93 | 367.72 | 129.67 | 90.00 | 0.00 | 53.16 | 78.63 |
| image002.jpg | 633.93 | 460.96 | 132.19 | 90.00 | 0.00 | 52.50 | 53.61 |
| image003.jpg | 633.93 | 554.20 | 128.95 | 90.00 | 0.00 | 54.05 | 51.71 |
| image004.jpg | 633.93 | 647.45 | 145.29 | 90.00 | 0.00 | 53.65 | 51.33 |
| image005.jpg | 633.93 | 740.69 | 137.78 | 90.00 | 0.00 | 54.10 | 52.41 |
| image006.jpg | 633.93 | 833.93 | 134.17 | 90.00 | 0.00 | 51.92 | 54.04 |
| image007.jpg | 733.93 | 367.72 | 110.75 | 90.00 | 0.00 | 54.57 | 51.58 |
| image008.jpg | 733.93 | 460.96 | 132.79 | 90.00 | 0.00 | 51.00 | 52.07 |
| image009.jpg | 733.93 | 554.20 | 115.80 | 90.00 | 0.00 | 53.62 | 52.20 |
| image010.jpg | 733.93 | 647.45 | 132.91 | 90.00 | 0.00 | 54.91 | 51.18 |
| image011.jpg | 733.93 | 740.69 | 151.41 | 90.00 | 0.00 | 54.13 | 51.79 |
| image012.jpg | 733.93 | 833.93 | 140.00 | 90.00 | 0.00 | 51.65 | 54.25 |
| image013.jpg | 833.93 | 367.72 | 125.77 | 90.00 | 0.00 | 53.24 | 74.50 |
| image014.jpg | 833.93 | 460.96 | 137.60 | 90.00 | 0.00 | 52.36 | 54.10 |
| Image015.jpg | 833.93 | 554.20 | 137.30 | 90.00 | 0.00 | 53.99 | 52.04 |
| Image016.jpg | 833.93 | 647.45 | 158.44 | 90.00 | 0.00 | 53.83 | 52.77 |
| Image017.jpg | 833.93 | 740.69 | 164.44 | 90.00 | 0.00 | 51.97 | 53.24 |
| Image018.jpg | 833.93 | 833.93 | 150.57 | 90.00 | 0.00 | 51.26 | 54.65 |

It is evident from the foregoing according to the invention that it is possible to detect a given portion of the body surface always in the same position and with the same inclination. To do so, in fact, it is provided that the subject is allowed to sit down in the following sessions always in the same position with respect to the above-mentioned reference anthropometrical points.

To this end, the invention provides a suitable control means for the correct repositioning of the subject which in the present embodiment illustrated by way of a not limiting example consists of laser beam projectors also performing the function of allowing the automatic focusing of the image collection means. In the embodiment illustrated in Figure 11, such laser projectors are placed at the upper and lower sides of such image collection means. According to the invention the repositioning of the tested subject is precise enough when the light beams produced by such laser projectors coincide to one spot at the predetermined repere point.

The present invention has been described and illustrated according to a preferred embodiment thereof, however, it is self-evident that anyone skilled in the art can make modifications and/or technically and functionally equivalent replacements without departing from the scope of the present industrial invention.

### Bibliography

[1]. Oliveira SA, Christos PJ et al.; Clin Epidemiol 1999 Nov; 52: 1111-6*.*
*[2].* Richard MA, Grob JJ et al.; Int J Cancer 2000 May; 20: 280-5*.*
[3]. Muhn CY et al.; J Am Acad Dermatol 2000 May; 42:754-9*.*
[4]. Hanrahan PF, Hersey et al: Arch Dermatol 1997 Mar; 133: 301-11*.*

## Claims

1. A method of detecting and showing variations in the number and/or the morphology of the external skin lesions of dermatological interest by taking a plurality of images of a subject, wherein in order to automate the detection and the transmission of said variations digital images of the body surface of the tested subject are collected after having divided the latter into one or more areas exactly located by means of spatial coordinates in a system of coordinated axes fixed with respect to predetermined reference points of the subject, the images being stored in a suitable database to then be compared automatically with corresponding images previously or subsequently collected, thus producing a signal of any variation in the number and/or the morphology/colour of the lesions; the relative spatial position between the subject body surface and the point of view from which said images are collected being the same for each subsequent corresponding image, wherein it is provided that each skin portion of the same subject is detected in subsequent times from a predetermined and fixed point of view , thereby obtaining that any variation of the collected images not relating to the state of the skin lesions is suppressed or minimized, **characterised in that** said image collecting means are positioned, using means for supporting and driving under control, perpendicularly to the area of the body surface to be detected and at constant distance therefrom.

2. The method according to the preceding claim, **characterized in that** there are provided the following operating steps:
A. subdividing the body surface into quadrants with suitable size;
B. selecting predetermined reference or "repere" anatomic points so that the following detection may have repere points able to match the body quadrants of the same subject;
C. collecting and storing high definition images relative to the above-mentioned quadrants;
D. processing the stored images to perform the following operations:
- locating, numbering and measuring all of the skin lesions present in each quadrant;
- storing images and data relative to said skin lesions;
- if the subject is not a new subject, comparing collected images and corresponding data with previously stored images and data of the same subject;
- highlighting and/or transmitting the new skin lesions in each quadrant and/or highlighting the morphological/colorimetric variations in one or more previously located skin lesions;
- storing data relative to the detected differences.

3. The method according to claim 1, **characterized in that** there are provided the following steps:
a) inputting anthropometrical data of the subject to be tested;
b) selecting the portion(s) of the body surface to be detected;
c) positioning the subject on the basis of the predetermined reference and/or repere points
d) calculating the coordinates of the centre of each image and the direction of collection of each of them;
e) collecting and storing said images automatically and repeatedly;
f) analysing the stored images to locate the existing skin lesions of interest;
g) comparing the analysed images with the images stored and analysed previously, if any, to highlight the presence of any numeric and/or colorimetric difference.

4. The method according to claim 2 or 3, **characterized in that** said processing or analysis of the stored images provides :
- locating objects contained in the image other than skin, e.g. underwear, background, etc.;
- locating structures that can produce false positives, e.g. hairs, spots produced by natural orifices or shadows, tattoos, etc.; and
- locating lesions of interest to be compared and ignoring objects and/or structures of the two preceding items.

5. The method according to the preceding claim, **characterized in that** the tested subject is allowed to sit down to the same position in any test following the first.

6. The method according to claim 1, **characterized in that** the body surface of the tested subject is segmented or sub-divided into images so that the edges of the images are partially overlapped so as to allow a comparison even when modifications of the body of the tested subject take place between subsequent tests.

7. The method according to the preceding claim, **characterized in that** the number of collected images for a determined patient is always the same, even if the patient increases in weight and/or height in time.

8. The method according to any preceding claim, **characterized in that** the patient is illuminated uniformly and from different angles so as to avoid shaded portions in the areas to be detected.

9. The method according to claim 3, **characterized in that** step f) relates to analysing images comprising a blue component and includes the following steps :
- recognizing not human pixels;
- removing piliferous appendages by parametrization software;
- detemining grey levels according to a weight of blue;
- constructing a background;
- constructing levels of identification of the pigmented areas;
- calculating mathematically the evidence threshold;
- recognizing the pigmented areas;
- characterizing the pigmented areas in terms of their specific qualities;
- differentiating the pigmented areas of the background noise, e.g. hair, underwear, tattoos, orifices, etc. objects.

10. The method according to claim 3, **characterized in that** step g) of image comparison includes the following steps:
- collimating frames;
- rotating/translating in scale;
- calculating the known connections;
- translating the pigmented areas, e.g. spot objects, to an assigned range to minimize the discards;
- calculating the differences, e.g. minus and/or plus dimensional - variation of the inner colour;
- optimising: if the number of erroneous connections is greater than three or if the secondary translation of the pigmented areas, e.g. spot objects, is greater than an assigned level, for example 100 pixels, a second collimation method is performed.

11. An apparatus for detecting images by the method according to the preceding claims, privided with:
a) an application software for processing fine graphic data, e.g. skin lesions, provided with algorithms able to provide a discrete set of the detected images;
b)a data base for the statistic analysis of data of interest;
c)a data processing portion for clinic, personal data of the subjects for storing and listing the images of each patient upon his/her visiting;
d)a reference surface provided with anthropometrical references with respect to which the tested subject is positioned;
e)means for lighting uniformly without shadows the zones of the subject body surface to be detected;
f)image collection means;
g)means for supporting and/or driving under control such image collection means with respect to the patient;
h)interface means for controlling the data collection and transmission to suitable storing and/or processing means;
i)at least a computer connected to such interface means;
j) at least a high definition monitor or video or other display means of the known type;
k)means for controlling the correct repositioning of the subject.
said means for supporting and/or driving said image collection means being controlled so as the relative spatial position of the subject body surface and the point of view from which the images are collected are the same for each subsequent corresponding image, **characterised in that** said support and drive means of the image collection means is able to position the latter perpendicular to the area of the body surface to be detected and at a constant distance therefrom.

12. The apparatus according to the preceding claim, **characterized in that** said anthropometrical references located in the reference surface for positioning the subject are able to locate the repere points which are significant for different somatic types as well as for the same patient subjected to several next detections so that defined, significant body areas that can be overlapped are obtained to guarantee a correct match of the collected images.

13. The apparatus according to the preceding claim, **characterized in that** it is provided with a computer which controls and manages the apparatus in a completely automatic way.

14. The apparatus according to claim 11, **characterized in that** it is provided with storing means which store the position and the orientation taken by said image collection means for each collected image, during a first session so that corresponding images can be collected in following sessions exactly with the same position and orientation, thus providing following images perfectly corresponding and comparable with those of the preceding session.

15. The apparatus according to claim 11, **characterized in that** it is provided with calculating means which calculate the positions of the images to be collected, so that the edges of images adjacent to one another are partially surmounted, thus forming an overlap.

16. The apparatus according to the preceding claim, **characterized in that** the overlap of the image edges adjacent to one another varies preferably from a maximum equal to half the height and half the width of the image to a minimum that can be zero, e.g. images with coinciding edges.

## Patentansprüche

1. Verfahren zum Detektieren und Darstellen von Änderungen der Anzahl und/oder der Morphologie der externen Hautläsionen von dermatologischem Interesse, bei dem eine Mehrzahl von Bildern einer Person aufgenommen wird, wobei zum Automatisieren der Detektion und Übertragung dieser Änderungen digitale Bilder der Körperoberfläche der Testperson erfasst werden, nachdem diese Körperoberfläche in einen oder mehrere Bereiche geteilt wurde, die mittels Raumkoordinaten in einem System von Koordinatenachsen fest und die bezüglich vorgegebener Referenzpunkte der Person exakt angeordnet sind, wobei die Bilder in einer geeigneten Datenbasis gespeichert werden, um dann automatisch mit entsprechenden zuvor oder später erfassten Bildern verglichen zu werden, wodurch ein Signal jeglicher Änderung der Anzahl und/oder der Morphologie/Farbe der Läsionen erzeugt wird; wobei die relative räumliche Position zwischen der Körperoberfläche der Person und dem Standpunkt, von dem aus die Bilder erfasst werden, für jedes spätere entsprechende Bild gleich ist, wobei vorgesehen ist, dass jeder Hautabschnitt desselben Person später aus einem vorgegebenen und festen Standpunkt detektiert wird, wodurch erreicht wird, dass jegliche Änderung der erfassten Bilder, die nicht mit dem Zustand der Hautläsionen zusammenhängt, unterdrückt oder minimiert wird, **dadurch gekennzeichnet, dass** die Bilderfassungsmittel unter Verwendung eines Mittels zur Halterung und zum gesteuerten Antrieb senkrecht zum Bereich der zu erkennenden Körperoberfläche und in einem konstanten Abstand davon positioniert werden.

2. Verfahren nach dem vorigen Anspruch, **dadurch gekennzeichnet, dass** die folgenden Verfahrensschritte vorgesehen sind:
A. Unterteilen der Körperoberfläche in Quadranten mit geeigneter Größe;
B. Wählen vorgegebener anatomischer Referenz- oder *"repère"-*Anhaltspunkte, so dass die folgende Detektion Anhaltspunkte haben kann, die mit den Körperquadranten derselben Person in Übereinstimmung gebracht werden kann;
C. Erfassen und Speichern hoch auflösender Bilder bezüglich der oben genannten Quadranten;
D. Verarbeiten der gespeicherten Bilder, um die folgenden Operationen auszuführen:
- Lokalisieren, Nummerieren und Vermessen aller in jedem Quadranten vorhandenen Hautläsionen;
- Speichern von Bildern und Daten bezüglich dieser Hautläsionen;
- wenn es sich bei der Person nicht um eine neue Person handelt, Vergleichen der erfassten Bilder und entsprechenden Daten mit zuvor gespeicherten Bildern und Daten derselben Person;
- Hervorheben und/oder Übertragen der neuen Hautläsionen in jedem Quadranten und/oder Hervorheben der morphologischen/kalorimetrischen Änderungen bei einer oder mehreren zuvor lokalisierten Hautläsionen;
- Speichern der Daten bezüglich der erkannten Differenzen.

3. Verfahren nach Anspruch 1,
**dadurch gekennzeichnet, dass** darin die folgenden Schritte vorgesehen sind:
a) Eingeben anthropometrischer Daten der zu testenden Person;
b) Auswählen des/der Abschnitts/Abschnitte der zu detektierenden Körperoberfläche;
c) Positionieren der Person auf Basis der vorgegebenen Referenz- und/oder Anhaltspunkte;
d) Berechnen der Koordinaten des Mittelpunktes jedes Bildes und der Richtung der Erfassungen jedes von ihnen;
e) automatisches und wiederholtes Erfassen und Speichern der Bilder;
f) Analysieren der gespeicherten Bilder, um die vorhandenen Hautläsionen von Interesse zu lokalisieren;
g) Vergleichen der analysierten Bilder mit den zuvor gespeicherten und analysierten Bildern, sofern vorhanden, um das Vorliegen jeglicher numerischer und/oder kalorimetrischer Unterschiede hervorzuheben.

4. Verfahren nach einem der Ansprüche 2 oder 3, **dadurch gekennzeichnet, dass** die Verarbeitung oder Analyse der gespeicherten Bilder vorsieht:
- Lokalisieren von anderen Objekten als Haut, die im Bild enthalten sind, z. B. Unterwäsche, Hintergrund usw.;
- Lokalisieren von Strukturen, die falsche positive Ergebnisse erzeugen können, z. B. Haare, Punkte aufgrund natürlicher Öffnungen oder Schatten, Tattoos usw.; und
- Lokalisieren von zu vergleichenden Läsionen von Interesse und Ignorieren von Objekten und/oder Strukturen gemäß den beiden vorangegangenen Punkten.

5. Verfahren nach dem vorigen Anspruch, **dadurch gekennzeichnet, dass** sich die getestete Person in jedem Test nach dem ersten in der gleichen Position setzen darf.

6. Verfahren nach Anspruch 1, **dadurch gekennzeichnet, dass** die Körperoberfläche der getesteten Person so segmentiert oder in Bilder unterteilt wird, dass die Ränder der Bilder teilweise überlappen, um einen Vergleich selbst dann zu ermöglichen, wenn sich Änderungen des Körpers der getesteten Person zwischen anschließenden Tests ergeben haben.

7. Verfahren nach dem vorigen Anspruch, **dadurch gekennzeichnet, dass** die Anzahl der erfassten Bilder für einen bestimmten Patienten immer gleich ist, selbst dann, wenn der Patient mit der Zeit an Gewicht und/oder Größe zunimmt.

8. Verfahren nach einem der vorigen Ansprüche, **dadurch gekennzeichnet, dass** der Patient gleichmäßig und aus verschiedenen Winkeln beleuchtet wird, um in den zu detektierenden Bereichen abgeschattete Abschnitte zu vermeiden.

9. Verfahren nach Anspruch 3, **dadurch gekennzeichnet, dass** Schritt f) die Analyse von Bildern betrifft, die eine blaue Komponente enthalten und die folgenden Schritte enthält:
- Erkennen nicht menschlicher Bildpunkte;
- Entfernen behaarter Anhangsgebilde durch eine Parametrisierungs-Software;
- Bestimmen von Graustufen gemäß der Gewichtung von Blau;
- Konstruieren eines Hintergrundes;
- Konstruieren von Identifikationsstufen der pigmentierten Bereiche;
- mathematisches Berechnen der Evidenzschwelle;
- Erkennen der pigmentierten Bereiche;
- Charakterisieren der pigmentierten Bereiche nach ihren spezifischen Eigenschaften;
- Differenzieren der pigmentierten Bereiche des Hintergrundrauschens, z. B. Haar-, Unterwäsche-, Tattoo-, Öffnungs- etc. -objekte.

10. Verfahren nach Anspruch 3, **dadurch gekennzeichnet, dass** Schritt g) des Bildvergleichs die folgenden Schritte enthält:
- Kollimieren von Einzelbildern;
- maßstäbliche Rotation/Translation;
- Berechnen der bekannten Verbindungen;
- Translation der pigmentierten Bereiche , z. B. Punktobjekte, zu einem zugeordneten Bereich, um Ausschuss zu minimieren;
- Berechnen der Differenzen, z. B. dimensionelle Abweichung der inneren Farbe nach minus und/oder plus;
- Optimieren: Wenn die Anzahl der fehlerhaften Verbindungen größer als drei ist, oder wenn die sekundäre Translation der pigmentierten Bereiche, z. B. Punktobjekte, größer ist als ein zugeordneter Pegel ist, z. B. 100 Bildpunkte, wird ein zweites Kollimationsverfahren ausgeführt.

11. Vorrichtung zum Erkennen von Bildern durch das Verfahren nach den vorigen Ansprüchen, mit:
a) einer Anwendungs-Software zum Verarbeiten feingraphischer Daten, z. B. Hautläsionen, die über Algorithmen verfügt, mit denen ein diskreter Satz der erkannten Bilder bereitgestellt werden kann;
b) einer Datenbasis für die statistische Analyse der Daten von Interesse;
c) einem Datenverarbeitungsabschnitt für klinische und persönliche Daten der Personen zum Speichern und Auflisten der Bilder jedes Patienten bei seinem Besuch;
d) einer Referenzoberfläche mit anthropometrischen Referenzen, bezüglich derer die getestete Person positioniert wird;
e) einem Mittel zum gleichmäßigen Beleuchten ohne Schatten der Zonen der zu detektierenden Körperoberfläche der Person;
f) einem Bilderfassungsmittel;
g) einem Mittel zum Haltern und/oder gesteuertem Antreiben eines solchen Bilderfassungsmittels bezüglich des Patienten;
h) einem Schnittstellenmittel zum Steuern der Datenerfassung und - übertragung an geeignete Speicher- und/oder Verarbeitungsmittel;
i) mindestens einem Computer, der an ein solches Schnittstellenmittel angeschlossen ist;
j) mindestens einem hoch auflösenden Monitor oder Videogerät oder einem anderen Anzeigemittel des bekannten Typs;
k) einem Mittel zum Steuern der korrekten Neupositionierung der Person;
wobei das Mittel zum Haltern und/oder gesteuerten Antreiben eines solchen Bilderfassungsmittels so gesteuert wird, dass die relative räumliche Position der Körperoberfläche der Person und der Standpunkt, von dem aus die Bilder erfasst werden, für jedes spätere entsprechende Bild gleich sind, **dadurch gekennzeichnet, dass** das Mittel zum Haltern und Antreiben des Bilderfassungsmittels in der Lage ist, das letztgenannte senkrecht zum Bereich der zu detektierenden Körperoberfläche und in einem konstanten Abstand zu dieser zu positionieren.

12. Vorrichtung nach dem vorigen Anspruch, **dadurch gekennzeichnet, dass** die anthropometrischen Referenzen, die sich in der Referenzoberfläche zum Positionieren der Person befinden, die Anhaltspunkte, die für verschiedene somatische Punkte signifikant sind, sowie für denselben Patienten, der verschiedenen nächsten Detektionen unterzogen wird, lokalisieren können, so dass definierte signifikante Körperbereiche, die überlappt sein können, erhalten werden, um eine korrekte Übereinstimmung der erfassten Bilder zu garantieren.

13. Vorrichtung nach dem vorigen Anspruch, **dadurch gekennzeichnet, dass** sie mit einem Computer ausgestattet ist, der die Vorrichtung auf eine vollkommen automatische Weise steuert und verwaltet.

14. Vorrichtung nach Anspruch 11, **dadurch gekennzeichnet, dass** sie mit Speichermitteln ausgestattet ist, die die Position und Ausrichtung des Bilderfassungsmittels für jedes erfasste Bild während einer ersten Sitzung speichern, so dass entsprechende Bilder in den folgenden Sitzungen mit exakt der gleichen Position und Ausrichtung erfasst werden können, so dass spätere Bilder bereitgestellt werden, die den der vorigen Sitzung exakt entsprechen und mit diesen vergleichbar sind.

15. Vorrichtung nach Anspruch 11, **dadurch gekennzeichnet, dass** sie mit Berechnungsmitteln ausgestattet ist, die die Positionen der zu erfassenden Bilder berechnen, so dass die Ränder benachbarter Bilder teilweise übereinander liegen und so eine Überlappung bilden.

16. Vorrichtung nach dem vorigen Anspruch, **dadurch gekennzeichnet, dass** die Überlappung der Ränder benachbarter Bilder vorzugsweise von einem Maximum gleich der Hälfte der Höhe und der Hälfte der Breite des Bildes bis zu einem Minimum, das null sein kann, d. h. Bilder mit zusammenfallenden Rändern, variiert.

## Revendications

1. Méthode de détection et d'affichage des variations du nombre et/ou de la morphologie de lésions cutanées externes présentant un intérêt dermatologique en prenant plusieurs images d'un sujet, dans laquelle, pour automatiser la détection et la transmission desdites variations, des images numériques de la surface corporelle du sujet testé sont recueillies après avoir divisé cette dernière en une ou plusieurs zones localisées exactement au moyen de coordonnées spatiales dans un système d'axes coordonnés fixes par rapport à des points de référence prédéterminés du sujet, les images étant stockées dans une base de données pour être ensuite comparées automatiquement à des images correspondantes antérieures ou recueillies par la suite, produisant ainsi un signal de toute variation en nombre et/ou morphologique/de couleur des lésions ; la position spatiale relative entre la surface corporelle du sujet et le point de vue duquel lesdites images sont recueillies étant la même pour chaque image correspondante consécutive, dans laquelle il est prévu que chaque partie de peau du même sujet soit détectée à des moments consécutifs d'un point de vue prédéterminé et fixe, ce qui permet que toute variation des images recueillies non apparentée à l'état des lésions cutanées soit supprimée ou minimisée, **caractérisée en ce que** lesdits moyens de recueil d'image sont positionnés en utilisant des moyens de soutien et de pilotage sous contrôle, perpendiculairement à la zone de la surface corporelle à détecter et à une distance constante de celle-ci.

2. Méthode selon la revendication précédente, **caractérisée en ce qu'**elle comprend les étapes de fonctionnement suivantes :
A. subdivision de la surface corporelle en quadrants présentant une taille appropriée ;
B. sélection de références prédéterminés ou de points anatomiques de "repère" de sorte que la détection suivante puisse avoir des points de repère pouvant s'apparier aux quadrants corporels du même sujet ;
C. recueil et stockage d'images de haute définition par rapport aux quadrants mentionnés ci-dessus ;
D. traitement des images stockées pour réaliser les opérations suivantes :
vocalisation, dénombrement et mesure de toutes les lésions cutanées présentes dans chaque quadrant ;
stockage des images et des données par rapport auxdites lésions cutanées ;
si le sujet n'est pas un nouveau sujet, comparaison des images recueillies et correspondance des données avec des images stockées précédemment et des données du même sujet ;
mise en évidence et/ou transmission des nouvelles lésions cutanées dans chaque quadrant et/ou mise en évidence des variations morphologiques/colorimétriques dans une ou plusieurs lésions cutanées localisées précédemment ;
stockage des données par rapport aux différences détectées.

3. Méthode selon la revendication 1, **caractérisée en ce qu'**elle comprend les étapes suivantes :
a) introduction de données anthropométriques du sujet à tester ;
b) sélection de la/des partie(s) de la surface corporelle à détecter ;
c) placement du sujet sur la base de la référence prédéterminée et/ou des points de repère,
d) calcul des coordonnées du centre de chaque image et de la direction de recueil de chacune d'elle ;
e) recueil et stockage desdites images automatiquement et de façon répétée ;
f) analyse des images stockées pour localiser les lésions cutanées existantes intéressantes ;
g) comparaison des images analysées avec les images stockées et analysées précédemment si elles existent, pour mettre en évidence la présence d'une différence numérique et/ou colorimétrique,

4. Méthode selon la revendication 2 ou 3, **caractérisée en ce que** ledit traitement ou ladite analyse des images stockées assure
la localisation des objets contenus dans l'image autre que la peau, par exemple, sous-vêtements, arrière-plan, etc. ;
la localisation des structures qui peuvent produire des résultats faussement positifs tels que poils, taches produites par des orifices naturels ou ombres, tatouages, etc. ;
la localisation des lésions intéressantes à comparer en ignorant les objets et/ou structures des deux items précédents.

5. Méthode selon la revendication précédente, **caractérisée en ce que** le sujet testé peut s'asseoir dans la même position dans tout test suivant le premier.

6. Méthode selon la revendication 1, **caractérisée en ce que** la surface corporelle du sujet testé est segmentée ou subdivisée en images de sorte que les bords des images soient partiellement chevauchants de façon à permettre une comparaison même si des modifications du corps du sujet testé surviennent entre les tests consécutifs.

7. Méthode selon la revendication précédente, **caractérisée en ce que** le nombre d'images recueillies pour un patient déterminé est toujours le même, même si le patient prend du poids ou grandit au cours du temps.

8. Méthode selon l'une quelconque des revendications précédentes, **caractérisée en ce que** le patient est éclairé uniformément et à partir d'angles différents de façon à éviter les parties d'ombre dans les zones à détecter.

9. Méthode selon la revendication 3, **caractérisée en ce que** l'étape f) concerne l'analyse d'images comprenant un composant bleu et comprend les étapes suivantes :
la reconnaissance de pixels non humains ;
l'élimination d'appendices pileux par un logiciel de paramétrage ;
la détermination des niveaux de gris par rapport à un poids de bleu ;
la construction d'un arrière-plan ;
la construction de niveaux d'identification des zones pigmentées ;
le calcul mathématique d'un seuil de mise en évidence ;
la reconnaissance des zones pigmentées ;
la caractérisation des zones pigmentées en termes de leurs qualités spécifiques ;
la différenciation des zones pigmentées du bruit de fond, par exemple, poils, sous-vêtements, tatouages, orifices, objets etc.

10. Méthode selon la revendication 3, **caractérisée en ce que** l'étape g) de la comparaison d'image comprend les étapes suivantes :
collimation de cadres ;
rotation/translation à l'échelle ;
calcul des connections connues ;
translation des zones pigmentées, par exemple taches d'objets à un ordre de grandeur assigné pour minimiser les chutes ;
calcul des différences, par exemple variations dimensionnelles supérieures et inférieures, variation de la couleur interne ;
optimisation : si le nombre de connections erronées est supérieur à trois ou si la translation secondaire des zones pigmentées, par exemple taches objets, est supérieur au niveau attribué, par exemple de 100 pixels, une deuxième méthode de collimation est réalisée.

11. Appareil de détection d'images par la méthode selon les revendications précédentes comportant :
a) un logiciel d'application pour traiter des données graphiques fines, par exemple, des lésions cutanées, doté d'algorithmes capables de fournir un groupe individuel d'images détectées ;
b) une base de données pour l'analyse statistique des données intéressantes ;
c) une partie de traitement de données pour des données cliniques personnelles des sujets pour le stockage et le listage des images de chaque patient après sa consultation ;
d) une surface de référence dotée de références anthropométriques par rapport auxquelles le sujet testé est placé ;
e) des moyens d'éclairage uniforme sans ombres des zones de la surface corporelle du sujet à détecter ;
f) des moyens de recueil d'image ;
g) des moyens de soutien et/ou de pilotage sous contrôle de ces moyens de recueil d'image par rapport au patient ;
h) des moyens d'interfaçage pour contrôler le recueil de données et la transmission à des moyens de stockage et/ou de traitement appropriés ;
i) au moins un ordinateur raccordé à ces moyens d'interfaçage ;
j) au moins un monitor haute définition ou une vidéo ou d'autres moyens d'affichage de type connu ;
k) des moyens de contrôle du repositionnement correct du sujet,
lesdits moyens de support et/ou de pilotage desdits moyens de recueil d'image étant commandés de sorte que la position spatiale relative de la surface corporelle su sujet et le point de vue duquel les images sont recueillies soient les mêmes pour chaque image correspondante consécutive, **caractérisée en ce que** lesdits moyens de support et de pilotage des moyens de recueil d'image peuvent positionner ces derniers perpendiculairement à la zone de surface corporelle à détecter et à une distance constante de celle-ci.

12. Appareil selon la revendication précédente, **caractérisé en ce que** lesdites références anthropométriques localisées sur la surface de référence pour positionner le sujet sont capables de localiser les points de repère qui sont significatifs pour différents types somatiques ainsi que pour le même patient soumis à plusieurs détections suivantes de sorte que des zones corporelles significatives définies qui peuvent se chevaucher sont obtenues pour garantir un appariement correct des images recueillies.

13. Appareil selon la revendication précédente, **caractérisé en ce qu'**il est doté d'un ordinateur qui commande et gère l'appareil de manière complètement automatique.

14. Appareil selon la revendication 11, **caractérisé en ce qu'**il est doté de moyens de stockage qui stockent la position et l'orientation prises par lesdits moyens de recueil d'image pour chaque image recueillie, pendant une première session de sorte que des images correspondantes puissent être recueillies dans les sessions suivantes exactement avec la même position et orientation, ce qui donne des images suivantes parfaitement correspondantes et comparables à celles de la session précédente.

15. Appareil selon la revendication 11, **caractérisé en ce qu'**il est doté de moyens de calcul qui calculent les positions des images à recueillir de sorte que les bords d'images adjacents les uns aux autres soient partiellement superposés, formant ainsi un chevauchement.

16. Appareil selon la revendication précédente, **caractérisé en ce que** le chevauchement des bords d'image adjacents les uns aux autres varie de préférence d'un maximum égal à la moitié de la hauteur et à la moitié de la largeur de l'image jusqu'à un minium qui peut être de zéro, par exemple, des images avec des bords coïncidents.
